# EUROPÄISCHE PATENTSCHRIFT

(11) **EP 0 466 974 B1**
(45) Veröffentlichungstag und Bekanntmachung des Hinweises auf die Patenterteilung: **18.01.1995**
(21) Anmeldenummer: 90124030.9
(22) Anmeldetag: 13.12.1990
(51) Int. Cl.: G01B 5/02, A61B 5/00

(54) **Einrichtung zum Erfassen der Umfangsform eines Körpers etwa elliptischen Querschnitts**
Device to measure the contour of a somewhat elliptical body
Dispositif pour mesurer le contour d'un corps quasi elliptique

(30) Priorität: 20.07.1990 DE 4023033
(43) Veröffentlichungstag der Anmeldung: 22.01.1992
(73) Patentinhaber: Manufacture Jaeger-Le Coultre S.A., CH-1347 Le Sentier (CH)
(72) Erfinder: Billmann, Reinhold, W-8507 Oberasbach (DE)
(74) Vertreter: Klein, Thomas, Dipl.-Ing. (FH)

(56) Entgegenhaltungen:
- AU-D- 6 900 574
- DE-A- 3 135 020
- PATENT ABSTRACTS OF JAPAN vol. 8, no. 55 (P-260), 13. März 1984 & JP-A-58 204 306

## Beschreibung

Die Erfindung bezieht sich auf eine Einrichtung und ein Verfahren zum Erfassen der Umfangsform eines Körpers etwa elliptischen Querschnitts, insbesondere der Umfangsform eines menschlichen Arms.

Soll ein Armreif oder eine aus zwei starren, jeweils mit einem Ende an einem Uhrgehäuse angelenkten Teilen gebildeten Armspannge hergestellt werden, ist es sinnvoll, daß eine Anpassung der Größe und Form an den Arm der vorgesehenen Trägerperson erfolgt.

Dies ist sehr aufwendig, da dazu die Trägerperson der der Armreif oder die Armspange angepaßt werden soll, dem die Anpassung vornehmenden Goldsschmied zur Verfügung stehen muß.

Insbesondere, wenn Trägerperson und Goldschmied sich an weit voneinander befindlichen Orten aufhalten, ist dies problematisch.

Aus "Patent Abstracts of Japan vol. 8, No. 55 (P-260) 13. März 1984 & JP-A-58204306 (Furuno Denkikk)" ist eine automatische Meß- und Anzeigevorrichtung für die Form eines Gegenstandes bekannt, die als Kette ausgebildet ist. Die Kettenglieder sind durch Stifte gelenkig miteinander verbunden. Durch ein Signalerfassungsteil werden die Verbindungspositonen der Kettenglieder zueinander erfaßt.

Aufgabe der Erfindung ist es, eine Einrichtung der eingangs genannten Art zu schaffen, die einfach aufgebaut ist und ein leichtes Erfassen der Umfangsform ermöglicht, sowie ein Verfahren anzugeben, durch das auf einfache Art die Umfangsform erfaßt wird.

Diese Aufgabe wird erfindungsgemäß bei einer Einrichtung der eingangs genannten Art durch mindestens zwei mit ihrer radial umlaufenden Innenkontur einen ellipsenartigen Ring bildenden Gliedern gelöst, die an ihren jeweils einander zugewandten freien Enden etwa in Umfangserstreckungsrichtung der Glieder relativ zueinander verschiebbar miteinander verbunden sind, wobei Markierungen auf den einander zugewandten freien Enden der Glieder in Umfangsersteckungsrichtung der Glieder hintereinander aufgebracht sind, mit denen das Maß der Verschiebung der Glieder aus einer Maximalposition heraus für jede Verbindungsstelle zweier Glieder meßbar ist.

Diese Einrichtung wird über den etwa elliptischen Körper, z. B. einen Arm geschoben und die Glieder so weit in die ringartige Innenkontur verkleinernde Richtung bewegt, bis sie an dem Arm zur Anlage kommen. Insbesondere, wenn eine größere Anzahl Glieder vorhanden ist, können auch Abweichungen von der idealen Ellipsenform weitgehend genau erfaßt werden.

Die Möglichkeit, die erfaßte Form zu definieren und zu beschreiben ist dadurch gegeben, daß das Maß der Verschiebung aus einer Maximalpositon heraus für jede Verbindungsstelle zweier Glieder gemessen wird. Dies kann z. B. anhand von Markierungen auf den einander zugewandten freien Enden der Glieder erfolgen, wobei die Markierungen in Umfangserstreckungsrichtung der Glieder hintereinander aufgebracht wird. Die dabei erfaßten Verschiebewerte der einzelnen ebenfalls definierten Verbindungsstellen können einer auch an entferntem Ort sich befindenden Person mitgeteilt werden, die an einem zweiten Exemplar der Einrichtung diese Werte einstellen und entsprechend einen Armreif anfertigen kann.

Die einander zugewandten freien Enden der Glieder können in mehreren Positionen relativer Verschiebungen zueinander feststellbar sein.

Die Positionen können dabei entweder stufenlos oder in Stufen äquidistanten Abstands einstellbar sein.

Ein Feststellen der Glieder in ihre Positionen kann entweder formschlüssig oder kraftschlüssig erfolgen.

Um ein Verschwenken der Glieder in ihrem Verbindungsbereich zu vermeiden, können die einander zugewandten freien Enden der Glieder formschlüssig aneinander geführt, relativ zueinander verschiebbar sein.

Eine Ausbildung der geführten Verschiebbarkeit besteht darin, daß die einander zugewandten freien Enden der Glieder teleskopartig relativ zueinander verschiebbar sind.

In einer anderen Ausbildung kann das eine der einander zugewandten freien Enden der Glieder mit einer sich in Umfangserstreckungsrichtung erstreckenden Führungsnut ausgebildet sein, in die das andere der freien Enden Glieder verschiebbar hineinragt. Dazu kann in einfacher Weise in einer Seitenwand der Führungsnut des einen freien Endes eines Gliedes eine sich in Umfangserstreckungsrichtung erstreckungsrichtung erstreckende Führungsrille ausgebildet sein, in die ein entsprechender an dem anderen freien Ende eines Glieds ausgebildeter Führungsansatz verschiebbar hineinragt.

Zur Festlegung der Einstellposition können quer zur Umfangserstreckungsrichtung in dem einen Ende am Bereich der Führungsnut und im Bereich des anderen in der Führungsnut verschiebbaren Endes in Überdeckung bringbare Ausnehmungen ausgebildet sein, in die ein Feststellelement einstellbar ist.

Ein selbsttätiges Lösen der Feststellelemente wird dabei dadurch verhindert, daß die Ausnehmungen in dem die Führungsnut aufweisenden Ende oder in dem in der Führungsnut verschiebbaren Ende Gewindebohrungen und das Feststellelement einer entsprechenden Schraube ist.

Auf leichte und sichere Art und Weise kann die Umfangsform eines Körpers etwa elliptischen Querschnitts, insbesondere die Umfangsform eines menschlichen Arms mittels einer Einrichtung nach Anspruch 1 nach folgendem Verfahren erfaßt werden,
- durch Aufschieben der Einrichtung auf den Körper derart, daß der Körper von der Einrichtung umschlossen ist,
- Verschieben der Glieder zueinander in die ellipsenartige Form der Innenkontur verringender Größe bis zum Anschlag an den Körper,
- Arretierung der Glieder in ihrer Anschlagposition und Definition jeder Verbindungsstelle zweier miteinander verbundener Gliederenden und Erfassung der Verschiebeposition der zwei Gliederenden relativ zueinander an jeder Verbindungsstelle durch Ablesen der jeweiligen Markierungen.

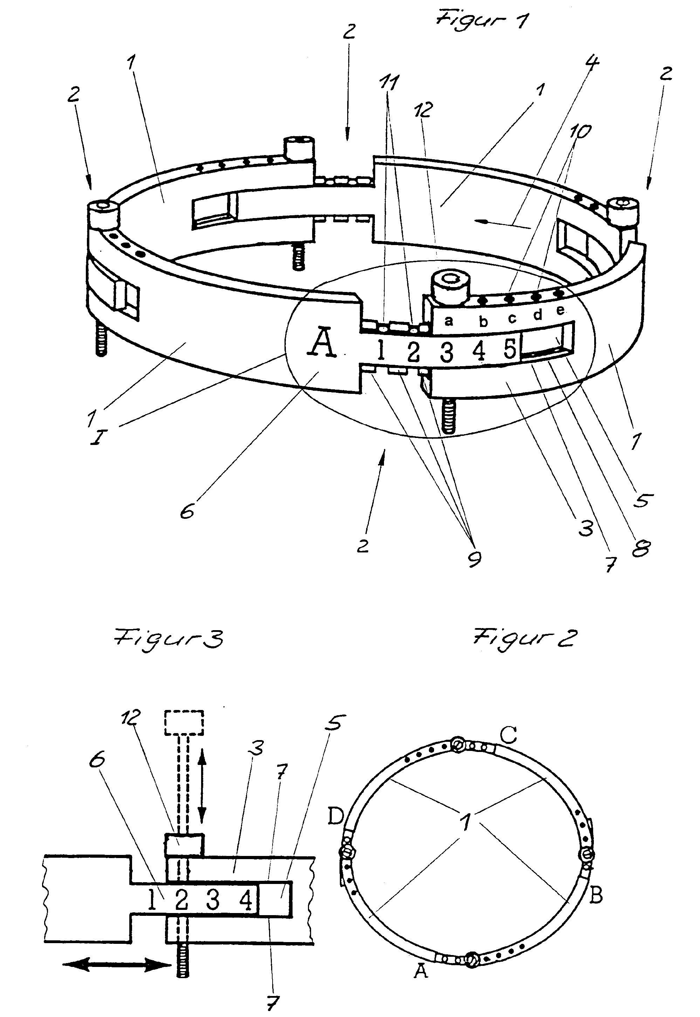

Ein Ausführungbeispiel der Erfindung ist in der Zeichnung dargestellt und wird im folgenden näher beschrieben. Es zeigen
- Figur 1: eine perspektivische Ansicht einer Einrichtung zum Erfassen der Umfangsform eines Körpers,
- Figur 2: eine Draufsicht der Einrichtung nach Figur 1,
- Figur 3: eine Seitenansicht des Ausschnitts I in Figur 1.

Die dargestellte Einrichtung besitzt vier Glieder 1 mit teilellipsenartiger Innenkontur, die an ihren einander zugewandten freien Enden derart zu einem Ring miteinander verbunden sind, daß ihre Gesamt-Innenkontur einen ellipsenartigen Ring bildet.

Jede Verbindungsstelle 2 zweier miteinander verbundener Gliederenden ist so ausgebildet, daß das Ende 3 des einen Glieds 1 mit einer sich in Umfangserstreckungsrichtung 4 des Rings sich erstreckenden Führungsnut 5 ausgebildet ist, die zu dem Ende 6 des anderen Glieds dieser Verbindungsstelle 2 hin offen ist.

Das Ende 6 ist auf die Breite der Führungsnut 5 verjüngt, sich in Umfangserstreckungsrichtung erstreckend ausgebildet, wobei dieses Ende 6 verschiebbar in der Führungsnut 5 angeordnet ist.

In den beiden Seitenwänden 7 der Führungsnut 5 sind in Umfangserstreckungsrichtung 4 sich erstreckende Führungsrillen 8 ausgebildet, in die entsprechende, an dem verjüngten Ende 6 ausgebildete Führungsansätze 9 verschiebbar hineinragen.

Im Bereich der Führungsnut 5 und im Bereich des in die Führungsnut 5 hineinragenden Bereichs des Endes 6 sind quer zur Umfangserstreckungsrichtung durchgehende Ausnehmungen 10 und 11 ausgebildet. Dabei bilden sowohl die Ausnehmungen 10 als auch die Ausnehmungen 11 jeweils in eine sich in Umfangserstreckungsrichtung hintereinander mit bestimmtem Abstand angeordnete Reihen. Je nach dem Grad der relativen Verschiebung der beiden Enden 3 und 6 zueinander sind bestimmte Ausnehmungen 10 mit bestimmten Ausnehmungen 11 in Überdeckung bringbar. In diese in Überdeckung gebrachten Ausnehmungen 10 und 11 ist als Feststellelement eine Schraube 12 einsetzbar, wobei der auf einer Seite der Führungsnut 5 befindliche Bereich der Ausnehmung 11 mit einem entsprechenden Gewinde ausgebildet ist.

Jede Verbindungsstelle 2 ist mit einer Bezeichnung ABC bzw. D versehen. Weiterhin sind die Ausnehmungen 11 mit Zahlen und die Ausnehmungen 10 mit den Buchstaben abcd und e bezeichnet. Nach Anlegen der Einrichtung an einem Arm und Zusammenschieben der Glieder 1 bis zur Anlage am Arm kann für jede Verbindungsstelle die Verschiebeposition bestimmt werden, indem die Buchstaben und Zahlen der in Überdeckung gebrachten Ausnehmungen 10 und 11 festgestellt werden. Damit kann auch einer an einem entfernten Ort befindlichen Person, die eine gleiche Einrichtung besitzt, eine exakte Information über die Form und Größe des gemessenen Arms übermittelt werden.

## Patentansprüche

1. Einrichtung zum Erfassen der Umfangsform eines Körpers etwa elliptischen Querschnitts, insbesondere der Umfangsform eines menschlichen Armes, mit mindestens zwei mit ihrer radial umlaufenden Innenkontur einen ellipsenartigen Ring bildenden Gliedern (1), die an ihren jeweils einander zugewandten freien Enden (3, 6) etwa in Umfangserstreckungsrichtung (4) der Glieder (1) relativ zueinander verschiebbar miteinander verbunden sind, wobei Markierungen auf den einander zugewandten freien Enden der Glieder (1) in Umfangserstreckungsrichtung der Glieder (1) hintereinander aufgebracht sind, mit denen das Maß der Verschiebung der Glieder (1) aus einer Maximalpositon heraus für jede Verbindungsstelle zweier Glieder (1) meßbar ist.

2. Einrichtung nach Anspruch 1, dadurch gekennzeichnet, daß die einander zugewandten freien Enden (3, 6) der Glieder (1) in mehreren Positionen relativer Verschiebung zueinander feststellbar sind.

3. Einrichtung nach Anspruch 2, dadurch gekennzeichnet, daß die Positionen stufenlos einstellbar sind.

4. Einrichtung nach Anspruch 2, dadurch gekennzeichnet, daß die Positionen in Stufen äquidistanten Abstands einstellbar sind.

5. Einrichtung nach einem der vorhergehenden Ansprüche, dadurch gekennzeichnet, daß die Glieder (1) formschlüssig feststellbar sind.

6. Einrichtung nach einem der Ansprüche 1 bis 4, dadurch gekennzeichnet, daß die Glieder kraftschlüssig feststellbar sind.

7. Einrichtung nach einem der vorhergehenden Ansprüche, dadurch gekennzeichnet, daß die einander zugewandten freien Enden (3, 6) der Glieder (1) formschlüssig aneinander geführt und relativ zueinander verschiebbar sind.

8. Einrichtung nach Anspruch 7, dadurch gekennzeichnet, daß die einander zugewandten freien Enden (3, 6) der Glieder (1) teleskopartig relativ zueinander verschiebbar sind.

9. Einrichtung nach Anspruch 7, dadurch gekennzeichnet, daß das eine der einander zugewandten freien Enden (3, 6) der Glieder (1) mit einer sich in Umfangserstreckungsrichtung (4) erstreckenden Führungsnut (5) ausgebildet ist, in die das andere der freien Enden (3, 6) der Glieder (1) verschiebbar hineinragt.

10. Einrichtung nach Anspruch 9, dadurch gekennzeichnet, daß in einer Seitenwand (7) der Führungsnut (5) des einen freien Endes (3) eines Gliedes (1) eine sich in Umfangserstreckungsrichtung (4) erstreckende Führungswelle (8) ausgebildet ist, in die ein entsprechender an dem anderen Ende (6) eines Glieds (1) ausgebildeter Führungsansatz (9) verschiebbar hineinragt.

11. Einrichtung nach Anspruch 9, dadurch gekennzeichnet, daß quer zur Umfangserstreckungsrichtung (4) in dem einen Ende (3) im Bereich der Führungsnut (5) und im Bereich des anderen in der Führungsnut (5) verschiebbaren Endes (6) in überdeckung bringbare Ausnehmungen (10 und 11) ausgebildet sind, in die ein Feststellelement einsetzbar ist.

12. Einrichtung nach Anspruch 11, dadurch gekennzeichnet, daß die Ausnehmungen (10) in dem die Führungsnut (5) aufweisenden Ende (3) oder in dem in der Führungsnut (5) verschiebbaren Ende (6) Gewindebohrungen und das Feststellelement eine entsprechende Schraube (12) ist.

13. Verfahren zum Erfassen der Umfangsform eines Körpers etwa elliptischen Querschnitts, insbesondere der Umfangsform eines menschlichen Arms, mittels einer Einrichtung nach Anspruch 1 mit folgenden Schritten,
- Aufschieben der Einrichtung auf den Körper derart, daß der Körper von der Einrichtung umschlossen ist,
- Verschieben der Glieder (1) zueinander in die ellipsenartige Form der Innenkontur verringernder Größe bis zum Anschlag an dem Körper,
- Arretierung der Glieder (1) in ihrer Anschlagposition und Definition jeder Verbindungsstelle (2) zweier miteinander verbundener Gliederenden (3, 6) und Erfassung der Verschiebeposition der beiden Gliederenden (3, 6) relativ zueinander an jeder Verbindungsstelle (2) durch Ablesen der jeweiligen Markierungen.

## Claims

1. A device for determining the circumferential shape of a body of approximately elliptical cross-section, particularly the circumferential shape of a human arm, having at least two members (1) which form an ellipse-like ring with their radially encircling internal contour and which are joined to each other at their mutually facing free ends (3, 6) in each case so that they are displaceable in relation to each other approximately in the direction of the circumferential span (4) of the members (1), wherein markings on the mutually facing free ends of the members (1) are provided one behind another in the direction of the circumferential span of the members (1), by means of which markings the extent of displacement of the members (1) from a maximum position can be measured for each junction point of two members (1).

2. A device according to claim 1, characterised in that the mutually facing free ends (3, 6) of the members (1) can be fixed in a plurality of positions of relative displacement to each other.

3. A device according to claim 2, characterised in that the positions are continuously adjustable.

4. A device according to claim 2, characterised in that the positions are adjustable in steps of equidistant intervals.

5. A device according to any one of the preceding claims, characterised in that the members (1) can be fixed with positive locking.

6. A device according to claims 1 to 4, characterised in that the members can be fixed with non-positive locking.

7. A device according to any one of the preceding claims, characterised in that the mutually facing free ends (3, 6) of the members (1) are guided with positive locking on each other and are displaceable in relation to each other.

8. A device according to claim 7, characterised in that the mutually facing free ends (3, 6) of the members (1) are telescopically displaceable in relation to each other.

9. A device according claim 7, characterised in that one of the mutually facing free ends (3, 6) of the members (1) is constructed with a guide slot (5) extending in the direction of the circumferential span (4), into which guide slot the other free end (3, 6) of the members (1) displaceably projects.

10. A device according to claim 9, characterised in that a guide flute (8), which extends in the direction of the circumferential span (4), is formed in a sidewall (7) of the guide slot (5) of one free end (3) of a member (1), into which guide flute a correspond guide projection (9) formed on the other end of the member (1) displaceably projects.

11. A device according to claim 9, characterised in that recesses (10 and 11), which can be brought into superposition and in which a fixing element can be inserted, are formed transverse to the direction of the circumferential span (4) in one end (3) in the region of the guide slot (5) and in the region of the other end (6) which is displaceable in the guide slot (5).

12. A device according to claim 11, characterised in that the recesses (10) in the end (3) comprising the guide slot (5) or in the end (6) which is displaceable in the guide slot (5) are tapped holes and the fixing element is a corresponding screw (12).

13. A method for determining the circumferential shape of a body of approximately elliptical cross-section, particularly the circumferential shape of a human arm, by means of a device according to claim 1, comprising the following steps:
- sliding the device on to the body so that the body is surrounded by the device,
- displacing the members (1) in relation to each other into the ellipsoidal shape of an internal contour of decreasing size until they contact the body,
- locking the members (1) in their contact position and defining each junction point (2) of two member ends (3, 6) connected to each other, and determining the position of displacement of the two member ends (3, 6) in relation to each other at each junction point (2) by observing the respective markings.

## Revendications

1. Dispositif pour détecter la forme périphérique d'un corps ayant une section droite à peu près elliptique, en particulier la forme du contour d'un bras humain, comprenant au moins deux éléments (1) formant par leur contour intérieur, faisant tout le tour radialement, un anneau semblable à une ellipse, qui sont reliés entre eux à leurs extrémités libres (3, 6) dirigées chaque fois l'une vers l'autre, de manière qu'ils soient déplaçables l'un par rapport à l'autre, notamment en translation, à peu près dans la direction d'extension périphérique (4) des éléments (1), des repères étant apposés les uns derrière les autres dans la direction d'extension périphérique des éléments (1) sur les extrémités libres, dirigées l'une vers l'autre,des éléments (1), repères par lesquels peut être mesurée, pour chaque point de jonction de deux éléments (1), la grandeur du déplacement des éléments (1) à partir d'une position maximale.

2. Dispositif selon la revendication 1, caractérisé en ce que les extrémités libres (3, 6), dirigées l'une vers l'autre, des éléments (1) sont blocables l'une par rapport à l'autre en plusieurs positions de déplacement relatif.

3. Dispositif selon la revendication 2, caractérisé en ce que les positions sont réglables de manière continue.

4. Dispositif selon la revendication 2, caractérisé en ce que les positions sont réglables par paliers équidistants.

5. Dispositif selon une des revendications précédentes, caractérisé en ce que les éléments (1) sont blocables mutuellement par une liaison obtenue par complémentarité de formes.

6. Dispositif selon une des revendications 1 à 4, caractérisé en ce que les éléments sont blocables mutuellement par une liaison obtenue par l'action d'une force.

7. Dispositif selon une des revendications précédentes, caractérisé en ce que les extrémités libres (3, 6), dirigées l'une vers l'autre, des éléments (1) sont guidées l'une sur l'autre par complémentarité de formes et mobiles en translation l'une par rapport à l'autre.

8. Dispositif selon la revendication 7, caractérisé en ce que les extrémités libres (3, 6), dirigées l'une vers l'autre, des éléments (1) sont déplaçables télescopiquement l'une par rapport à l'autre.

9. Dispositif selon la revendication 7, caractérisé en ce que l'une des extrémités libres (3, 6), dirigées l'une vers l'autre, des éléments (1) est pourvue d'une rainure de guidage (5) orientée dans la direction d'extension périphérique (4), rainure dans laquelle pénètre et peut glisser l'autre des extrémités libres (3, 6) des éléments (1).

10. Dispositif selon la revendication 9, caractérisé en ce qu'un flanc latéral (7) de la rainure de guidage (5) d'une extrémité libre (3) d'un élément (1) présente une cannelure de guidage (8) orientée dans la direction d'extension périphérique (4), cannelure dans laquelle pénètre et peut glisser un appendice de guidage (9) complémentaire formé sur l'autre extrémité (6) d'un élément (1).

11. Dispositif selon la revendication 9, caractérisé en ce que des évidements (10 et 11), orientés transversalement à la direction d'extension périphérique (4), sont ménagés respectivement dans une extrémité (3), au droit de la rainure de guidaqge (5), et dans l'autre extrémité (6), disposée coulissante dans cette rainure (5), évidements qui peuvent être amenés à coïncider et dans lesquels peut être introduit un organe de blocage.

12. Dispositif selon la revendication 11, caractérisé en ce que les évidements (10) de l'extrémité (3) pourvue de la rainure de guidage (5), ou de l'extrémité (6) disposée coulissante dans cette rainure (5), sont des trous taraudés, et l'organe de blocage est une vis (12) correspondante.

13. Procédé pour déceler la forme périphérique d'un corps ayant une section droite à peu près elliptique, en particulier la forme du contour d'un bras humain, au moyen d'un dispositif selon la revendication 1, comprenant les étapes suivantes:
- glissement du dispositif sur le corps, de manière que le corps soit entouré par le dispositif,
- déplacement des éléments (1) les uns par rapport aux autres, dans le sens de la diminution de la grandeur de la forme semblable à une ellipse du contour intérieur, jusqu'à application des éléments contre le corps, et
- immobilisation des éléments (1) à leur position d'application contre le corps et définition de chaque point de liaison (2) de deux extrémités d'éléments (3, 6) reliées l'une à l'autre, ainsi que détermination de la position de déplacement des deux extrémités d'éléments (3, 6) l'une par rapport à l'autre à chaque point de jonction (2) par lecture des repères conjugués.
